# EUROPEAN PATENT SPECIFICATION

(11) **EP 2 148 232 B1**
(45) Date of publication and mention of the grant of the patent: **20.05.2015**
(21) Application number: 09009375.8
(22) Date of filing: 20.07.2009
(51) Int. Cl.: G02B 7/02, G02B 23/24, A61B 1/12, A61B 1/00

(54) **Endoscope optical sytem device and endoscope with the same**
Optische Endoskopsystemvorrichtung und Endoskop damit
Dispositif de système optique d'endoscope et endoscope doté de celui-ci

(30) Priority: 22.07.2008 JP 2008188186
(43) Date of publication of application: 27.01.2010
(73) Proprietor: FUJIFILM Corporation, Minato-ku Tokyo (JP)
(72) Inventor: Takahashi, Kazuaki, Saitama-shi Saitama (JP); Kitano, Ryou, Saitama-shi Saitama (JP); Yashito, Takashi, Saitama-shi Saitama (JP); Yamamoto, Goki, Saitama-shi Saitama (JP)
(74) Representative: Klunker . Schmitt-Nilson . Hirsch

(56) References cited:
- JP-A- 2010 069 096
- US-A- 5 894 369
- US-A1- 2002 010 385
- US-B1- 6 239 922
- US-B1- 6 370 422
- US-B2- 6 565 505
- US-B2- 6 695 775

## Description

### BACKGROUND OF INVENTION

### Field of the Invention

The present invention relates to an endoscope optical system device according to the preamble of claim 1, and an endoscope with the same.

### Description of the Related Art

An endoscope used to observe/inspect a body cavity, or the like is equipped with an illuminating unit and an observing unit at an inserting portion that is inserted into the body cavity, or the like. The illuminating unit has a light guide for transmitting an illumination light emitted from a light source device. An illumination lens, from which the illumination light fed from the light guide is emergent, is fitted to a top end of the inserting portion. The observing unit has an objective optical system that is used to pick out image information in the body cavity to the outside, and displays the video image in the body cavity on a monitor device, or the like. The objective optical system consists of plural sheets of lenses. During the inspection, sometimes contaminants such as body fluids, or the like adhere to the lens, which is arranged at the top end of the inserting portion to expose to the outside, and thus the observation visual field becomes poor. Therefore, the contaminants are washed away by injecting wash water to the lens from a nozzle.

In accordance with the preamble of claim 1, US 6,239,922 discloses an endoscope optical system device (figure 6) in which the first and second optical element are received in a frame member and are bonded to the frame member.

US 6,370,422 B1 discloses a fiber-optical imaging apparatus which makes use of a coupling lens. The coupling lens comprises plural lens elements which are first glued to their respective mounts with a first adhesive, followed by applying epoxy to the lenses for greater attachment toughness and durability.

US 6,695,775 B2 discloses an endoscope in which an adhesive material is used in combination with solder in order to prevent fogging.

The inserting portion is employed during the inspection in a state that this portion is warmed up to about 40 to 50 °C by a heat generated from the solid-state imaging element, the light guide, etc. When the wash water whose temperature is low at about 10 to 20 °C is injected to an outer surface of the lens whose temperature is increased, the cooling of the lens is caused quickly. Thus, when moisture has entered into the inside of the objective lens, the formation of condensation occurs on the lens and is adhered onto the inner surface side of the lens. Therefore, such a problem existed that a picture quality of the observed video image deteriorates.

In order to prevent such formation of condensation on the lens, various proposals have been made (for example, see JP-A-61-107210, JP-A-5-281492). As shown in FIG.6, in an endoscope 1 set forth in JP-A-61-107210, a top end cover lens 9 and a filter 11 are arranged into a top-end cover lens containing recess 5 and a filter containing recess 7 both provided to a top constituent member 3 respectively, and an adhesive 13 is filled into clearances between outer peripheral surfaces of the top end cover lens 9 and the filter 11 and the containing recesses 5, 7 to secure them together. Accordingly, airtightness of the top end portion can be ensured and also entering of the moisture into the inside can be prevented.

Also, as shown in FIG.7, an optical system device 21 set forth in JP-A-5-281492 is equipped with a first optical element 25 and a second optical element 27 in a lens frame 23. The first optical element 25 and the second optical element 27 are joined hermetically at joined surfaces 29 by the joining means (adhesive). Accordingly, an air layer S formed between the first optical element 25 and the second optical element 27 can be closed in an airtight state, and thus entering of the moisture into the air layer S can be prevented.

However, in the endoscope 1 set forth in JP-A-61-107210, a layer of the adhesive 13 is provided merely between an outer peripheral surface of the top end cover lens 9 and an inner peripheral surface of the top-end cover lens containing recess 5 and between an outer peripheral surface of the filter 11 and an inner peripheral surface of the filter containing recess 7.
Therefore, it is possible that the moisture, particularly, a water molecule passes through the layer of the adhesive 13 and enters into the inside. Also, in the optical system device 21 set forth in JP-A-5-281492, it is only the joining means (adhesive) for adhering the first optical element 25 and the second optical element 2 at the joined surfaces 29 that seals hermetically the air layer S in an airtight state. Therefore, it is impossible to say that the sufficient hermetic sealing can always be kept by merely adhering these narrow joined surfaces 29, and sometimes the moisture is ready to enter into the air layer S. Also, in some cases the above approach cannot be applied to adhere the moisture-resistant optical element whose moisture permeability is low but which cannot be used because of its low adhesive strength.

### SUMMARY OF INVENTION

The present invention has been made in view of the above circumstances and it is an object of the present invention to provide an endoscope optical system device capable of preventing fogginess of an optical element caused due to a formation of condensation without fail by increasing airtightness of a top end portion of an endoscope, and an endoscope with the same.

(1) According an aspect of the invention , an endoscope optical system device includes an optical system which is arranged on a top end side of an inserting portion of an endoscope and in which a plurality of optical elements are aligned, and the device includes a first optical element which is arranged closest to a side of an observation object; and a second optical element which is joined hermetically to the first optical element via an air layer; the device being characterized by a first adhesive which joins a joined surface between the first and second optical elements; and a second adhesive which covers a periphery of end portions of the joined surface, wherein moisture permeability of the second adhesive is lower than that of the first adhesive.

In the endoscope optical system device constructed in this manner, the joined surfaces of the first optical element and the second optical element are adheres by the first adhesive, and peripheries of end portions of the joined surfaces are covered with the second adhesive with a moisture resistance. Therefore, such an event can be prevented without fail that moisture seeps in the joined boundary from the outside via the peripheries of the end portions of the joined surfaces and then enters into the air layer that is formed between the first optical element and the second optical element. As a result, the formation of condensation on the lens can be prevented in advance, and also a reduction in picture quality of the observed video image can be prevented.

Also, the adhesives that are excellent in each feature respectively are employed separately such that an adhering strength can be ensured by the first adhesive and also a moisture resistance can be ensured by the second adhesive whose moisture permeability is low. Therefore, the optical system device having a high airtightness and a high adhering strength can be constructed.

(2) In the endoscope optical system device of (1), the first optical element includes a plano-concave lens, having a concave portion on one side facing to the second optical element.

In the endoscope optical system device constructed in this manner, it prevents the air layer between the concave portion of the plane-concave lens of the first optical lens and the second optical lens from entering moisture from the outside of the endoscope. Thus, it is prevented from fogging caused by dew condensation of the plane-concave lens.

(3) In the endoscope optical system device of (1) or (2), the second optical element includes a parallel plate.

In the endoscope optical system device constructed in this manner, it becomes simple assembly steps, since it is unnecessary to adjust the position such as optical axis due to the parallel plate of the second optical element when the first optical lens joins the second optical lens.

(4) In the endoscope optical system device of (1) to (3), an outer diameter of the second optical element is smaller than an outer diameter of the first optical element, and
the second adhesive is coated on a stepped portion which is formed by a difference between the outer diameter of the first and second optical elements in a case that the first and second optical elements are joined together.

In the endoscope optical system device constructed in this manner, the second adhesive whose moisture permeability is low is coated on the stepped portion formed when an outer diameter of the second optical element is set smaller than an outer diameter of the first optical element.
Therefore, it can be prevented substantially that the external moisture passes through the second adhesive. As a result, such an event can be prevented that the moisture seeps in the air layer that is interposed between the first optical element and the second optical element. Also, the second adhesive never squeezes out outward in the diameter direction, and the optical system device can be constructed in compact.

(5) The endoscope optical system device of (1) to (4) further may includes: a lens holder which is arranged with a outer periphery of the plurality of the optical elements, wherein the lens holder holds the optical elements so that a closed space is formed between an inner peripheral surface of the lens holder and the first and second optical elements, and the second adhesive is coated to fill the closed space.

In the endoscope optical system device constructed in this manner, the second adhesive is coated to fill the closed space that is formed between the first and second optical elements and the lens holder. Therefore, the moisture passing path can be restricted by shielding an outer air. As a result, such an event can be prevented more surely that the moisture seeps in the air layer that is interposed between the first optical element and the second optical element.

(6) In the endoscope optical system device of (1) to (5), the optical system guides a light to an imaging element that fetches image information.

In the endoscope optical system device constructed in this manner, the optical system is the optical system that guides a light to the imaging element that fetches the image information. Therefore, the image in the body cavity can be converted into the electric signal and then output to the external device, and can be displayed on a monitor device, or the like.

(7) In the endoscope optical system device of (1) to (6), the optical system emits an illumination light.

In the endoscope optical system device constructed in this manner, the optical system is the optical system that emits an illumination light. Therefore, a desired part in the body cavity can be illuminated brightly, and the clear image in the body cavity can be picked up.

(8) In the endoscope optical system device of (1) to (7), the moisture permeability of the second adhesive is set to 9 g/m²/24h or less.

In the endoscope optical system device constructed in this manner, a moisture permeability of the second adhesive is set to 9 g/m²/24h or less. Therefore, the sure moisture proof effect can be achieved.

(9) An endoscope includes an endoscope optical system device according to (1) to (8) is provided to a top end side of an inserting portion of the endoscope.

According to the endoscope, the bright and clear image can be obtained.

According to the endoscope optical system device and the endoscope with the same, the joined surfaces of the first optical element and the second optical element are adhered together by the first adhesive, and peripheries of end portions of the joined surfaces are covered with the second adhesive with a moisture resistance. Therefore, such an event can be prevented without fail that the moisture seeps in the joined boundary from the outside via the peripheries of the end portions of the joined surfaces and then enters into the air layer that is formed between the first optical element and the second optical element. As a result, the formation of condensation on the lens can be prevented in advance, and also a reduction in picture quality of the observed video image can be prevented.

### BRIEF DESCRIPTION OF THE DRAWINGS

FIG.1 is an external perspective view showing a top end portion of an inserting portion of an endoscope;
FIG. 2 is a sectional view of a lens device of the endoscope shown in FIG.1;
FIG. 3 is a longitudinal sectional view showing a configuration of an objective optical system;
FIG. 4A is a pertinent enlarged view showing a state that a first optical element and a second optical element both having the same outer diameter are joined together;
FIG. 4B is a pertinent enlarged view showing a state that a second optical element having a larger outer diameter is joined to a first optical element together;
FIG. 5 is a pertinent sectional view of an optical system device applied to an illumination unit;

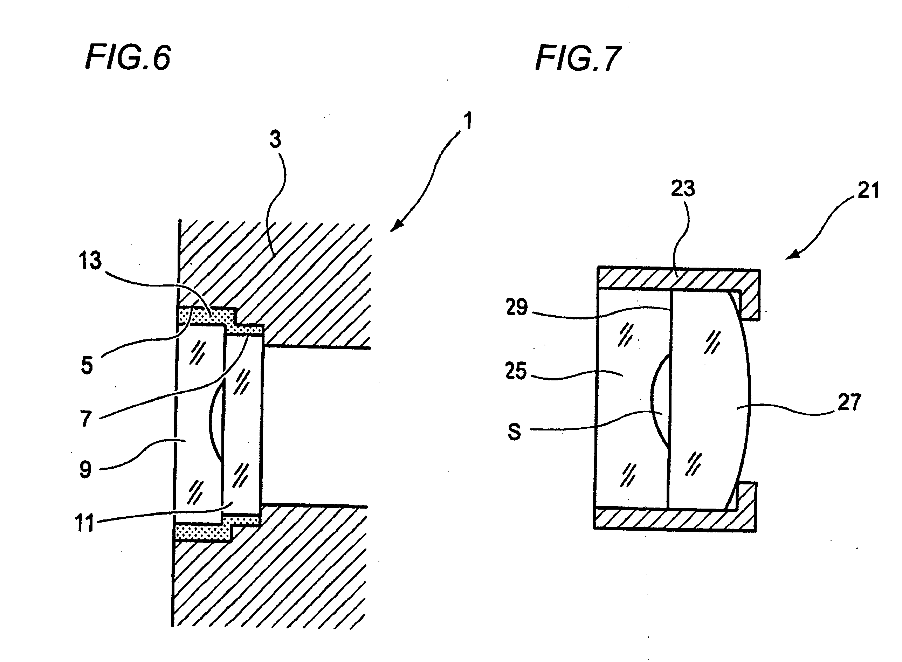

FIG. 6 is a pertinent sectional view of an optical system device in the related art; and
FIG. 7 is a pertinent sectional view of another optical system device in the related art.

### DESCRIPTION OF EXEMPLARY EMBODIMENTS

Embodiments of an optical system device of an endoscope and an endoscope with the same according to the present invention will be explained in detail with reference to the drawings hereinafter.

### (First Embodiment)

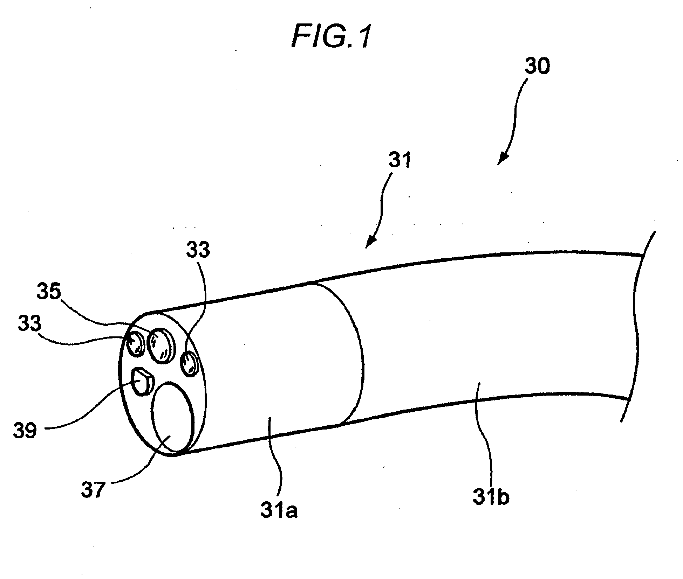

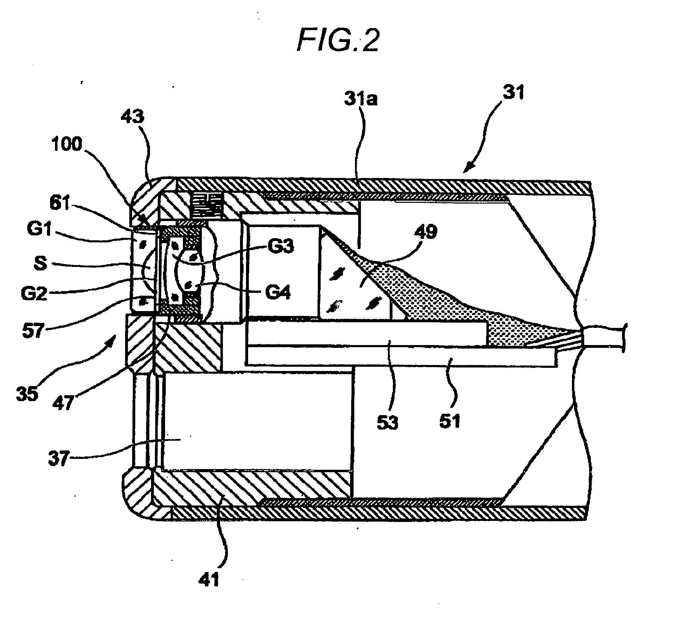

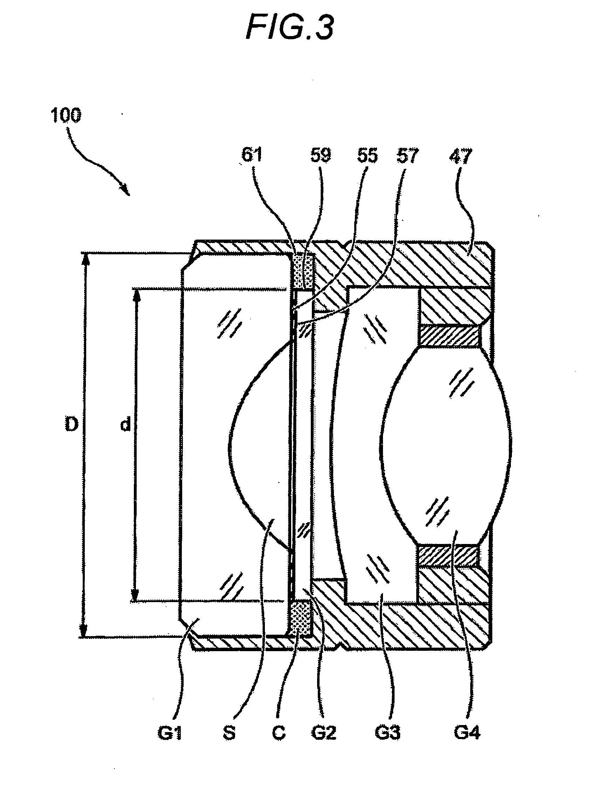

FIG.1 is an external perspective view showing a top end portion of an inserting portion of an endoscope, FIG.2 is a sectional view of a lens device of the endoscope shown in FIG.1, and FIG.3 is a longitudinal sectional view showing a configuration of an objective optical system.

As shown in FIG.1 to FIG.3, an inserting portion 31 of an endoscope 30 inserted in the body cavity, or the like has a top end hard portion 31a whose top end portion is formed of a hard material. An angle portion 31b that can operate the top end hard portion 31a in a desired direction while being bent is connected at the back of the top end hard portion 31 a. An illumination window 33 and an observation window 35 are provided to a top end surface (although not shown, a side surface of a laterally viewing type endoscope) of the top end hard portion 31 a, and a treating-tool passing channel 37 for passing a treating tool such as a forceps, or the like through there is opened on the top end surface. Also, a nozzle 39 for injecting a washing fluid, e.g., the wash water and a pressurized air, toward the observation window 35 when the observation window 35 is soiled is provided.

As shown in FIG.2, the top end hard portion 31a is constructed by a top end portion main body 41 and a top end cap 43. The top end portion main body 41 is formed of a metal in which plural through holes are formed to pass through in an axial direction. The top end cap 43 is made of an electrical insulating member that covers an outer surface of the top end portion main body 41 to prevent such an event that the metal is exposed directly to the outside. Also, joined portions of the top end portion main body 41 and the top end cap 43 are sealed with the sealing member.

An observe unit 100 as an optical system device provided to the observation window 35 is constructed by incorporating an objective optical system into a lens holder 47. In order to fix the lens holder 47 to the top end hard portion 31 a, a through hole that passes through from the top end cap 43 to the top end portion main body 41 in the axial direction is formed. An objective optical system consisting of a plurality of lenses, described later, as the optical element is fitted into the lens holder 47. Also, a prism 49 for turning an optical axis of the objective optical system at a right angle is joined/fixed to an end surface of the lens holder 47. Also, a solid state imaging element 53 as an imaging unit fitted to a substrate 51 is joined to the prism 49.

Next, an example of the objective optical system provided to the lens holder 47 is shown in FIG.3. The objective optical system shown in FIG.3 is constructed to have four sheets of lenses, i.e., first to fourth lenses G1, G2, G3, and G4 are aligned in sequence from the optical element arranged on the outermost side (observation object side) out of a plurality of optical elements.

The first optical element G1 is formed as a plano-concave lens whose observation object side is shaped into a planar surface and whose focusing side (a side facing to the second optical element G2) is shaped into a concave surface, and the planar surface is exposed to the observation window 35. Also, the second optical element G2 positioned closer to the focusing side than the first optical element G1 is formed as the parallel plate. The second optical element G2 is joined hermetically to the first optical element (lens) G1 (in FIG.3, a thickness of the adhesive layer is illustrated in an exaggerated manner). Accordingly, an air layer S is formed between the first optical element (lens) G1 and the second optical element (parallel plate) G2.

Also, the third optical element G3 is formed as a convexo- concave lens whose concave surface is directed to the focusing side, and the fourth optical element G4 is formed as a convex lens. In this case, the third optical element (lens) G3 and the fourth optical element (lens) G4 constitute a cemented lens.

The first lens G1 and the parallel plate G2 are adhered firmly at joined surfaces 55 by a first adhesive 57 whose adhesive strength is high, and are joined hermetically together. Also, an outer diameter D of the first lens G 1 is larger than an outer diameter d of the parallel plate G2. When the first lens G1 and the parallel plate G2 are joined together, a stepped portion 59 is formed on outer peripheries of end portions of the joined surfaces 55. When the first lens G1 and the parallel plate G2 are fitted into the lens holder 47, a closed space C is formed between an inner peripheral surface of the lens holder 47 and the first lens G1 and the parallel plate G2, and a second adhesive 61 whose moisture permeability is lower than the first adhesive 57 is coated on a full circumference of the closed space C. The a second adhesive 61 may be coated closely to cover at least a space between the first lens G1 and the parallel plate G2. The moisture resistance can be improved much more by providing the second adhesive to fill the closed space C.

The fitting operation of the optical elements G 1, G2, G3 and G4 into the lens holder 47 will be made as follows. That is, the first lens G1 and the parallel plate G2 are adhered at the joined surfaces 55 by the first adhesive 57, and also the second adhesive 61 is coated on the stepped portion 59. Then, the first lens G1 and the parallel plate G2 are inserted into the lens holder 47 from the front surface side (the left side in FIG.3), and are secured to the lens holder 47 by applying the caulking using the roller. In this event, because the second optical element is the parallel plate G2, there is no necessity that alignments of the optical axis, and the like should be made strictly during the adhesion to the first lens G1, so that the assembling steps can be simplified. Also, the third lens G3 and the fourth lens G4 are inserted into the lens holder 47 from the rear surface side (the right side in FIG.3), then secured by the adhesive, and then fitted to the lens holder 47.

Both biocompatibility and chemical resistance are required of the first adhesive 57 that is used in the endoscope 30. As the first adhesive 57 whose main purpose is to adhere the first lens G1 and the parallel plate G2 firmly, an epoxy- based adhesive such as CS2340-5 (manufactured by Cemedine Co., Ltd), or the like, for example, is illustrated. A moisture permeability of the first adhesive 57 is set in a range of 45 to 90 g/m²/24h (based on JIS Z0208).

Also, as the second adhesive 61 whose main purpose is to prevent a moisture, a fluorine-based adhesive with a low moisture permeability such as X-71-708 (manufactured by Shin-Etsu Chemical Co., Ltd: moisture permeability is 9 g/m²/24h (after cured at 23 °C/55 %RH/7 days)), or the like, for example, and an organic polymer-based adhesive such as a polyolefin- based resin, or the like may be listed. The second adhesive 61 may have the moisture permeability of 9 g/m²/24h or less. Although the concrete illustration is omitted, it was understood by experiment that, when the moisture permeability is about 15 g/m²/24h, the influence of moisture permeation begins to appear. The certain moisture resistant effect could be achieved by the adhesive whose moisture permeability is 9 g/m²/24h. In this case, because the outside of the second adhesive 61 is covered with the first adhesive 57, biocompatibility is not required of the second adhesive 61 and a margin of choice of the adhesive material can be enhanced.

The observe unit 100 is fitted to the top end hard portion 31a such that the lens holder 47 is inserted into the through hole provided from the top end portion main body 41 to the top end cap 43.

Next, an action of the observe unit (optical system device) 100 will be explained hereunder.

When the moisture is contained in the air layer S between the first lens G 1 and the parallel plate G2, there is a possibility that the formation of condensation occurs. The first lens G1 is exposed to the observation window 35, and further the wash water is injected onto the surface from the nozzle 39. When the wash water is injected, the inner surface of the first lens G1, particularly the thinnest part of the lens, is cooled quickly by the wash water. As a result, the formation of condensation occurs intensively on the center thinnest portion on the inner surface side of the first lens G1, which may be not in obtaining the clear endoscope image.

For this reason, it is requested that the moisture should not be contained in the air layer S between the first lens G1 and the parallel plate G2. Also, it is requested that the air layer S should be sealed perfectly and held in such a state that no moisture enters into this air layer from the outside for a long term.

In the optical system device 100 of the endoscope 30, the first lens G1 and the parallel plate G2 are adhered at the joined surfaces 55 by the first adhesive 57 whose adhesive strength is high, and the second adhesive 61 whose moisture resistance is high (whose moisture permeability is low) is coated on the stepped portion 59 on the peripheries of the end portions of the joined surfaces 55. Therefore, the moisture that came from the outside can be blocked by the second adhesive 61 whose moisture resistance is high, and thus such an event can be prevented without fail that the moisture enters into the air layer S between the first lens G1 and the parallel plate G2. As a result, the formation of condensation on the first lens G1 can be prevented in advance, and also a reduction in picture quality of the observed video image can be prevented.

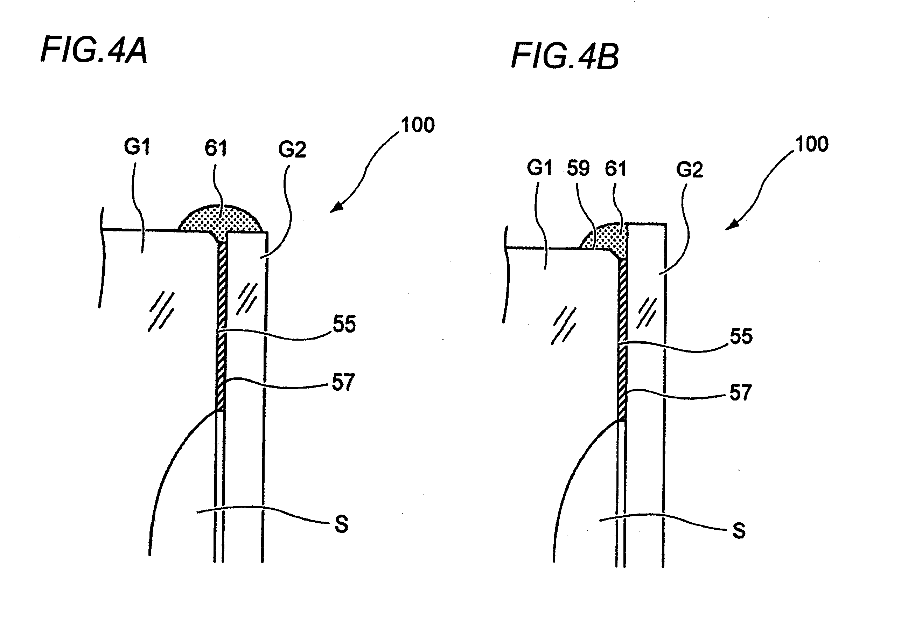

Next, a variation of the observe unit (optical system device) 100 will be explained with reference to FIG.4 hereunder. FIG.4A is a pertinent enlarged view showing a state that the first lens G1 and the parallel plate G2 both having the same outer diameter are joined together, and FIG.4B is a pertinent enlarged view showing a state that the parallel plate G2 having the larger outer diameter is joined to the first lens G1 together.

As shown in FIG.4A, the first lens G1 and the parallel plate G2 both having the same outer diameter are adhered at the joined surfaces 55 by the first adhesive 57 whose adhesive strength is high, and the second adhesive 61 whose moisture resistance is high is coated on the peripheries of the end portions of the joined surfaces 55. Also, as shown in FIG.4B, the parallel plate G2 having the larger outer diameter than that of the first lens G1 is adhered at the joined surfaces 55 by the first adhesive 57 whose adhesive strength is high, and the second adhesive 61 whose moisture resistance is high is coated on the stepped portion 59 formed on the peripheries of the end portions of the joined surfaces 55.

Other operations and advantages are similar to those in the optical system device 100 in the first embodiment, and therefore their explanation will be omitted herein.

### (Second Embodiment)

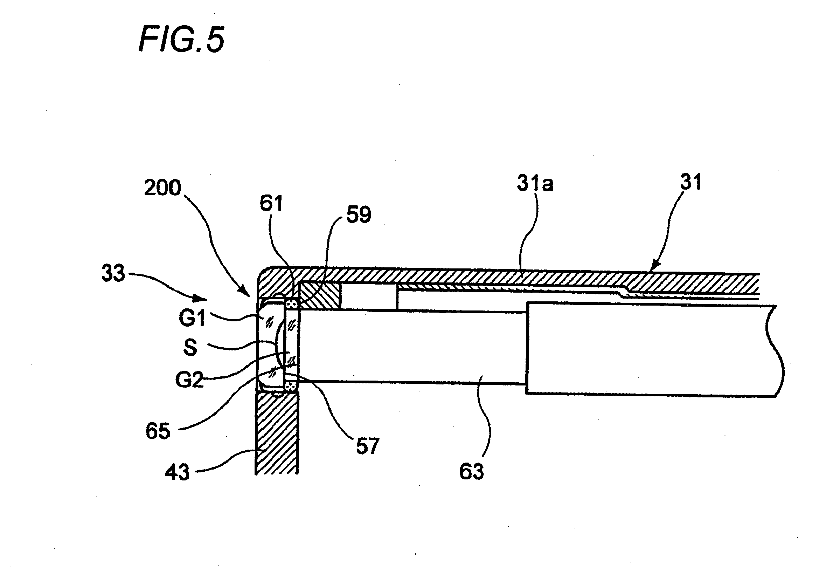

Next, an optical system device according to a second embodiment will be explained with reference to FIG.5 hereunder. FIG.5 is a pertinent sectional view of an optical system device applied to an illumination unit as an illuminating unit. In this case, in the optical system device according to the second embodiment, an optical element arranged at the back of the parallel plate (second optical element) is different from the optical element in the first embodiment. Remaining elements are similar to the optical system device in the first embodiment, and therefore their explanation will be omitted or simplified by affixing the same or equivalent reference symbols to the same portions.

As shown in FIG.5, in an illumination unit 200 as an optical system device in the second embodiment, the first lens G1 and the parallel plate G2 are arranged in the illumination window 33 that is provided to the top end of the inserting portion 31. Like the optical system device in the first embodiment, the first lens G1 and the parallel plate G2 are adhered at the joined surfaces 55 by the first adhesive 57 whose adhesive strength is high, and the second adhesive 61 whose moisture resistance is high is coated on the peripheries of the end portions of the joined surfaces 55.

An emergent end 65 of a light guide 63 is positioned at the back (the right side in FIG.5) of the parallel plate G2. The other end of the light guide 63 is connected to a light source device (not shown). An illumination light from a light source lamp is transmitted through the light guide 63, and is emergent from the illumination window 33 via the illumination unit 200.

When the moisture enters into the air layer S between the first lens G1 and the parallel plate G2 from the outside, in some cases the formation of condensation occurs on the first lens G1 and thus a luminous intensity of the illumination unit 200 is decreased. In the illumination unit 200 in the present embodiment, the first lens G1 and the parallel plate G2 are adhered at the joined surfaces 55 by the first adhesive 57 whose adhesive strength is high, and the second adhesive 61 whose moisture resistance is high (whose moisture permeability is low) is coated on the stepped portion 59 on the peripheries of the end portions of the joined surfaces 55. Therefore, the moisture that came from the outside can be blocked by the second adhesive 61 whose moisture resistance is high. As a result, the formation of condensation on the first lens G1 can be prevented in advance, and also the illumination of high luminous intensity can be provided.

Here, the optical system device of the present endoscope is not limited to the embodiments and the variation mentioned above, and variations, improvements, etc. can be applied appropriately. For example, in the above description, the explanation is made on the assumption that the parallel plate is employed as the second optical element. In this case, an infrared cut filter required when the solid state imaging element may be employed or another lens can be employed.

## Claims

1. An endoscope optical system device that includes an optical system which is arranged on a top end side of an inserting portion of an endoscope and in which a plurality of optical elements are aligned, the device comprising:
a first optical element (G1) which is arranged closest to a side of an observation object; and
a second optical element (G2) which is joined hermetically to the first optical element via an air layer (5);
**characterized by**
a first adhesive (57) which joins a joined surface between the first and second optical elements (G1, G2); and
a second adhesive 61 which covers a periphery of end portions of the joined surface,
wherein moisture permeability of the second adhesive (61) is lower than that of the first adhesive (57).

2. The endoscope optical system device according to claim 1, wherein the first optical element (G1) includes a plano-concave lens having a concave portion on one side facing to the second optical element (G2).

3. The endoscope optical system device according to claim 1 or 2, wherein the second optical element (G2) includes a parallel plate.

4. The endoscope optical system device according to any one of claims 1 to 3, wherein an outer diameter of the second optical element (G2) is smaller than an outer diameter of the first optical element (G1), and
the second adhesive (61) is coated on a stepped portion which is formed by a difference between the outer diameter of the first and second optical elements in a case that the first and second optical elements are joined together.

5. The endoscope optical system device according to any one of claims 1 to 4, further comprising:
a lens holder (47) which is arranged with a outer periphery of the plurality of the optical elements (G1-G4),
wherein the lens holder (47) holds the optical elements so that a closed space (G) is formed between an inner peripheral surface of the lens holder and the first and second optical elements, and
the second adhesive (61) is coated to fill the closed space.

6. The endoscope optical system device according to any one of claims 1 to 5, wherein the optical system guides a light to an imaging element that fetches image information.

7. The endoscope optical system device according to any one of claims 1 to 6, wherein the optical system emits an illumination light.

8. The endoscope optical system device according to any one of claims 1 to 7, wherein the moisture permeability of the second adhesive is set to 9 g/m²/24h or less.

9. An endoscope, comprising:
an endoscope optical system device according to any one of claims 1 to 8 is provided to a top end side of an inserting portion of the endoscope.

## Patentansprüche

1. Optische Endoskopvorrichtung, enthaltend ein optisches System, welches an einem Vorderende eines Einführabschnitts eines Endoskops angeordnet ist, und in welchem mehrere optische Elemente ausgerichtet sind, umfassend:
ein erstes optisches Element (G1), welches am nächsten auf einer Seite eines Beobachtungsobjekts angeordnet ist; und
ein zweites optisches Element (G2), das hermetisch mit dem ersten optischen Element über eine Luftschicht (S) vereint ist;
**gekennzeichnet ist, durch**
einen ersten Klebstoff (57), der eine Verbindungsfläche zwischen dem ersten und dem zweiten optischen Element (G1, G2) vereint; und
einen zweiten Klebstoff (61), der eine Peripherie der Endbereiche der Verbindungsfläche abdeckt,
wobei die Feuchtigkeits-Durchlässigkeit des zweiten Klebstoffs (61) geringer ist als diejenige des ersten Klebstoffs (57).

2. Optische Endoskopvorrichtung nach Anspruch 1, bei der das erste optische Element (G1) eine plan-konkave Linse mit einem konkaven Abschnitt auf einer dem zweiten optischen Element (G2) zugewandten Seite enthält.

3. Optische Endoskopvorrichtung nach Anspruch 1 oder 2, bei der das zweite optische Element (G2) eine Parallelplatte enthält.

4. Optische Endoskopvorrichtung nach einem der Ansprüche 1 bis 3, bei der ein Außendurchmesser des zweiten optischen Elements (G2) kleiner ist als ein Außendurchmesser des ersten optischen Elements (G1), und
der zweite Klebstoff (61) auf einen abgestuften Abschnitt aufgetragen ist, der gebildet ist durch eine Differenz zwischen dem Außendurchmesser des ersten und des zweiten optischen Elements für den Fall, dass das erste und das zweite optische Element zusammengefügt sind.

5. Optische Endoskopvorrichtung nach einem der Ansprüche 1 bis 4, weiterhin umfassend:
einen Linsenhalter (47), der an einem Außenumfang der mehreren optischen Elemente (G1-G4) angeordnet ist,
wobei der Linsenhalter (47) die optischen Elemente derart hält, dass zwischen der Innenumfangsfläche des Linsenhalters und dem ersten und dem zweiten optischen Element ein geschlossener Raum (C) gebildet ist, und
der zweite Klebstoff (61) aufgetragen ist, um den geschlossenen Raum zu füllen.

6. Optische Endoskopvorrichtung nach einem der Ansprüche 1 bis 5, bei der das optische System Licht zu einem bildgebenden Element leitet, welches Bildinformation aufnimmt.

7. Optische Endoskopvorrichtung nach einem der Ansprüche 1 bis 6, bei der das optische System ein Beleuchtungslicht emittiert.

8. Optische Endoskopvorrichtung nach einem der Ansprüche 1 bis 7, bei der die Feuchtigkeits-Durchlässigkeit des zweiten Klebstoffs auf 9 g/m²/24h oder weniger eingestellt ist.

9. Endoskop, umfassend:
eine optische Endoskopvorrichtung nach einem der Ansprüche 1 bis 8, angeordnet an einem Vorderende eines Einführabschnitts des Endoskops.

## Revendications

1. Dispositif de système optique d'endoscope qui inclut un système optique agencé sur un côté d'extrémité supérieur d'une partie d'introduction d'un endoscope et dans lequel une pluralité d'éléments optiques sont alignés, le dispositif comprenant :
un premier élément optique (G1) agencé le plus près d'un côté d'un objet d'observation, et
un second élément optique (G2) joint de manière hermétique au premier élément optique via une couche d'air (5) ;
**caractérisé par**
un premier adhésif (57) qui joint une surface jointe entre les premier et second éléments optique (G1, G2), et
un second adhésif (61) qui recouvre une périphérie de parties d'extrémité de la surface jointe,
dans lequel une perméabilité à l'humidité du second adhésif (61) est inférieure à celle du premier adhésif (57).

2. Dispositif de système optique d'endoscope selon la revendication 1, dans lequel le premier élément optique (G1) inclut une lentille plan-concave, présentant une partie concave sur un côté faisant face au second élément optique (G2).

3. Dispositif de système optique d'endoscope selon la revendication 1 ou 2, dans lequel le second élément optique (G2) inclut une plaque parallèle.

4. Dispositif de système optique d'endoscope selon l'une quelconque des revendications 1 à 3, dans lequel un diamètre extérieur du second élément optique (G2) est inférieur à un diamètre extérieur du premier élément optique (G1), et
le second adhésif (61) est appliqué sur une partie étagée qui est formée par une différence entre le diamètre extérieur des premier et second éléments optiques dans un cas où les premier et second éléments optiques sont joints ensemble.

5. Dispositif de système optique d'endoscope selon l'une quelconque des revendications 1 à 4, comprenant en outre :
un support de lentille (47) agencé avec une périphérie extérieure de la pluralité des éléments optiques (G1-G4) ;
dans lequel le support de lentille (47) maintient les éléments optiques de telle sorte qu'un espace fermé (G) est formé entre une surface périphérique interne du support de lentille et les premier et second éléments optiques, et
le second adhésif (61) est appliqué pour remplir l'espace fermé.

6. Dispositif de système optique d'endoscope selon l'une quelconque des revendications 1 à 5, dans lequel le système optique guide une lumière vers un élément d'imagerie qui extrait des informations d'image.

7. Dispositif de système optique d'endoscope selon l'une quelconque des revendications 1 à 6, dans lequel le système optique émet une lumière d'éclairement.

8. Dispositif de système optique d'endoscope selon l'une quelconque des revendications 1 à 7, dans lequel la perméabilité à l'humidité du second adhésif est réglée sur 9 g/m²/24 h ou moins.

9. Endoscope comprenant :
un dispositif de système optique d'endoscope selon l'une quelconque des revendications 1 à 8, prévu sur un côté d'extrémité supérieur d'une partie d'introduction de l'endoscope.
